# FASCICULE DE BREVET EUROPEEN

(11) **EP 4 132 633 B1**
(45) Date de publication et mention de la délivrance du brevet: **21.02.2024**
(21) Numéro de dépôt: 21720701.8
(22) Date de dépôt: 08.04.2021
(51) Int. Cl.: A61M 37/00

(54) **MODULE DE MARQUAGE POUR UN APPAREIL DESTINÉ À L'APPLICATION D'UN PRODUIT COLORANT ET APPAREIL UTILISANT UN TEL MODULE DE MARQUAGE**
MARKIERUNGSMODUL FÜR EIN GERÄT ZUM AUFTRAGEN EINES FÄRBEMITTELS UND GERÄT MIT SOLCH EINEM MARKIERUNGSMODUL
MARKING MODULE FOR AN APPLIANCE INTENDED FOR APPLYING A COLORANT, AND APPLIANCE USING SUCH A MARKING MODULE

(30) Priorité: 09.04.2020 CH 4352020
(43) Date de publication de la demande: 15.02.2023
(73) Titulaire: Armink Tattoo Body Art, 25370 Jougne (FR)
(72) Inventeur: KAUFFMANN, Steven, 1400 Yverdon-les-Bains VD (CH); REVOL, Vincent, 6023 Rothenburg LU (CH); VOLDEN, Tormod, 6003 Luzern LU (CH)
(74) Mandataire: Radzimski, Eric
(86) Numéro de dépôt international: PCT/EP2021/059236
(87) Numéro de publication internationale: WO 2021/204971

(56) Documents cités:
- WO-A1-2015/095904
- CA-A1- 3 090 248
- IT-A1-201800 007 613
- NL-B1- 2 020 030
- US-A1- 2020 023 175

## Description

### DOMAINE TECHNIQUE

La présente invention concerne le domaine du maquillage permanent ou du tatouage. Plus précisément, cette invention concerne un module de marquage pour un appareil destiné à l'application d'un produit colorant pour le maquillage permanent ou le tatouage, ce module de marquage comportant un canal et au moins une aiguille mobile longitudinalement dans ledit canal entre une position rétractée dans laquelle une extrémité distale de l'aiguille se trouve à l'intérieur dudit canal et une position étendue dans laquelle l'extrémité distale de l'aiguille dépasse dudit canal.

L'invention concerne également un appareil pour l'application d'un produit colorant pour le maquillage permanent ou le tatouage.

### ART ANTÉRIEUR

Actuellement, les appareils destinés à l'application d'un produit colorant sur une personne et utilisés pour le maquillage permanent ou le tatouage sont formés d'un pistolet dans lequel une aiguille de tatouage se déplace longitudinalement, et d'un moteur destiné à déplacer cette aiguille. Lorsqu'un produit colorant tel qu'en particulier une encre doit être appliqué sur ou sous la peau d'un utilisateur, l'aiguille est trempée dans un réservoir d'encre, puis elle est appliquée sur la peau de l'utilisateur de façon à faire pénétrer l'encre sous la peau de l'utilisateur. Lorsque l'encre qui a été prélevée par l'aiguille est consommée, cette aiguille est de nouveau trempée dans le réservoir d'encre, puis le cycle recommence.

Un problème qui peut se produire avec ce genre d'appareils vient du fait que la quantité d'encre qu'il est possible d'utiliser entre chaque trempe dans le réservoir est relativement faible. Lorsque la personne qui effectue le tatouage doit par exemple tracer une ligne droite ou allongée, elle doit régulièrement arrêter le dessin pour reprendre de l'encre. La reprise du dessin à l'endroit exact où celui-ci a été arrêté est relativement difficile et il est possible que le dessin ne soit pas idéal pour cette raison.

Par ailleurs, la durée nécessaire pour effectuer un tatouage peut être longue à cause des recharges d'encres qu'il est nécessaire de réaliser régulièrement. Ceci est d'autant plus important notamment lorsque de grandes surfaces doivent être colorées, par exemple lors d'ombrages. Cette durée de travail relativement longue peut être désagréable pour la personne qui effectue le tatouage ou le maquillage, mais surtout pour la personne sur qui le tatouage ou le maquillage est effectué. En effet, le marquage sous la peau peut être relativement douloureux. Un temps de travail plus court amène donc plus de confort à la personne sur qui le marquage est effectué.

Un autre problème peut se produire du fait que les réservoirs contenant l'encre sont ouverts pour permettre le prélèvement d'encre. Cette encre se trouve donc en contact avec l'air. Il est alors possible qu'elle soit contaminée par des pathogènes qui peuvent ensuite être injectés sous la peau de la personne sur laquelle le tatouage ou le maquillage est effectué, ce qui peut engendrer des infections.

Pour répondre à certains de ces inconvénients, il existe un appareil de tatouage ou de maquillage permanent comportant un réservoir intégré au pistolet. Un tel appareil est décrit dans le brevet américain US 6,345,553. Ce document décrit un pistolet de tatouage ou de maquillage permanent comportant un module dans lequel une aiguille de tatouage se déplace longitudinalement sous l'impulsion d'un moteur. Le pistolet de tatouage comporte un réservoir d'encre. L'aiguille, lors de son déplacement longitudinal alternatif, se déplace dans le réservoir d'encre, puis son extrémité libre dépasse du pistolet de tatouage de façon à pouvoir appliquer l'encre prélevée par l'aiguille, sous la peau de l'utilisateur.

Cette réalisation évite en grande partie la recharge d'encre dans un réservoir externe. Il est donc possible de tracer des lignes relativement longues et de remplir des surfaces importantes sans devoir recharger de l'encre. De plus, l'encre n'est pas à l'air libre et les risques de contamination ou d'infections sont donc minimisés. Ce pistolet présente toutefois d'autres inconvénients. En effet, de l'encre est introduite dans le réservoir, puis l'aiguille se déplace dans ce réservoir. Il n'est pas possible de vider l'encre du réservoir et de la remplacer par une encre de couleur différente. En effet, ceci amènerait un mélange hétérogène de couleurs qui ne serait pas utilisable. Ainsi, si l'encre contenue dans le réservoir n'est pas totalement consommée, elle est perdue. Par ailleurs, le réservoir d'encre dans lequel se déplace l'aiguille se trouve dans une position relativement éloignée de l'extrémité libre de l'aiguille, c'est-à-dire de l'extrémité de laquelle l'aiguille débouche pour réaliser le tatouage. Pour que de l'encre puisse être prélevée par l'aiguille, il faut que son extrémité entre entièrement dans le réservoir. Ceci oblige à réaliser un guidage de l'aiguille particulièrement rigide, ce qui complique la réalisation du pistolet du point de vue mécanique et ce qui le renchérit. De plus, par la construction du pistolet, il est nécessaire que de l'encre soit disponible du côté du réservoir où débouche l'aiguille. Ceci oblige la personne qui effectue le tatouage à tenir le pistolet d'une certaine manière, l'aiguille sensiblement verticale pointant vers le bas, faute de quoi l'aiguille ne reçoit pas d'encre. Ceci rend ce pistolet peu pratique à utiliser.

Un autre inconvénient de ce dispositif vient du fait que l'aiguille baigne dans l'encre. Ceci implique que la seule encre qui peut être utilisée est celle contenue dans le réservoir. De ce fait, il n'est pas possible d'utiliser l'encre du réservoir pour une partie d'un tatouage, puis d'utiliser le dispositif en trempant l'aiguille dans de l'encre d'un réservoir externe pour réaliser une autre partie du tatouage.

Il en résulte que la solution décrite dans le brevet US 6,345,553 n'est pas idéale en pratique. En effet, il n'est pas possible de changer de couleur d'encre en conservant le même pistolet. L'encre non utilisée est perdue et la manipulation du pistolet doit se faire d'une certaine manière faute de quoi il ne fonctionne pas. De plus, il n'est pas possible d'utiliser le dispositif alternativement dans un mode « conventionnel » dans lequel l'aiguille est trempée dans un réservoir d'encre externe, et dans le mode utilisant l'encre contenue dans le réservoir intégré au dispositif.

Voir également WO2015/095904 A1.

Il existe donc un besoin pour un appareil de tatouage ou de maquillage permanent qui soit souple et pratique d'utilisation, c'est-à-dire en particulier qui permette une utilisation en continu sans devoir tremper l'aiguille dans de l'encre régulièrement, qui permette un changement de couleur de l'encre sans entraîner de gaspillage, qui puisse être utilisé dans les positions normales qu'une personne effectuant un tatouage utilise de façon conventionnelle et qui puisse être utilisé dans deux modes différents.

### DESCRIPTION DE L'INVENTION

Un tel appareil est décrit dans le présent document.

Les buts de l'invention sont atteints par un module de marquage tel que défini en préambule et caractérisé en ce qu'il comporte un réservoir de produit colorant et en ce que ledit réservoir communique avec ledit canal uniquement dans une zone située en aval de l'aguille lorsque cette aiguille se trouve dans ladite position rétractée.

Les buts de l'invention sont également atteints par un appareil pour l'application d'un produit colorant tel que défini en préambule et caractérisé en ce qu'il comporte un module de marquage tel que défini ci-dessus, et un module d'actionnement de l'aiguille du module de marquage.

Le module de marquage selon la présente invention peut être utilisé dans toutes sortes de positions, ce qui permet d'effectuer un maquillage permanent ou un tatouage sans que ni la personne qui effectue le tatouage ni celle qui le reçoit ne doivent être placées dans une position inconfortable.

Le tatouage peut être réalisé en un temps plus court qu'avec un appareil conventionnel, en particulier si le dessin est grand et/ou qu'il comporte de grandes surfaces à remplir. Cette diminution de la durée représente un confort aussi bien pour la personne qui effectue le marquage que pour celle sur qui le marquage est effectué.

De plus, avec le dispositif de l'invention, il est possible d'ajouter de l'encre de façon simple et pratique dans un réservoir, la quantité d'encre ajoutée correspondant aux besoins et non à la capacité du réservoir.

Il est également possible de changer la couleur de l'encre de façon simple, ce qui permet d'utiliser un même module de marquage pour plusieurs couleurs. Par ailleurs, il est possible d'utiliser des cartouches préremplies, ce qui évite des manipulations de l'encre. Il est également possible d'éviter que l'encre ne reste à l'air libre pendant une longue dure, ce qui évite les risques de contamination par un pathogène contenu dans l'air.

L'appareil de l'invention permet d'utiliser l'encre contenue dans la cartouche ou dans le réservoir intégré au dispositif. Il permet en outre de fermer le réservoir et d'utiliser le dispositif de façon conventionnelle, c'est-à-dire en trempant l'aiguille dans de l'encre contenue dans un réservoir externe.

Ceci est particulièrement intéressant dans le cas où une partie importante d'un tatouage ou d'un maquillage est réalisé en utilisant une couleur alors qu'une autre partie nécessite l'utilisation d'une autre couleur ou dans le cas où deux couleurs doivent être utilisées alternativement. Dans un tel cas, il n'est pas nécessaire de vider le réservoir intégré, de le nettoyer et de le remplir d'une autre couleur pour réaliser une partite partie du tatouage ou du maquillage. Il suffit de fermer le réservoir, puis d'utiliser le dispositif de façon conventionnelle, en prélevant de l'encre avec l'aiguille à partir d'un réservoir externe.

Lorsque cette partie du tatouage ou du maquillage est terminée, le réservoir peut être réouvert et l'encre provenant du réservoir intégré est de nouveau utilisée.

### BRÈVE DESCRIPTION DES DESSINS

La présente invention et ses avantages seront mieux compris en référence aux figures annexées et à la description détaillée d'un mode de réalisation particulier, dans lesquelles :
- la figure 1 illustre un module de marquage selon un premier mode de réalisation de la présente invention ;
- la figure 2 est une vue en coupe d'une partie du module de marquage de la figure 1, dans une première configuration;
- la figure 3 est une vue similaire à la figure 2, dans une deuxième configuration, l'aiguille étant dans une position rétractée ;
- la figure 4 est une vue similaire à la figure 3, l'aiguille étant dans une position avancée ;
- la figure 5 est une vue similaire à la figure 3, l'aiguille étant dans une position étendue ;
- la figure 6 est une vue en perspective de la partie du module de marquage de la figure 2 ;
- la figure 7 illustre un module de marquage selon un deuxième mode de réalisation de l'invention, dans lequel un réservoir d'encre est séparé du corps du module de marquage ;
- la figure 8 représente le module de marquage de la figure 7, le réservoir d'encre étant partiellement placé sur le corps de ce module de marquage ;
- la figure 9 représente le module de marquage de la figure 7, le réservoir d'encre étant placé sur le corps de ce module de marquage ;
- la figure 10 est une vue partiellement en coupe du module de marquage de la figure 7, de l'encre étant contenue dans le réservoir ;
- la figure 11 est une vue partiellement en coupe d'une cartouche amovible, dans une première configuration ;
- la figure 12 est une vue similaire à la figure 11, dans une deuxième configuration ;
- la figure 13 illustre un réservoir d'encre utilisé dans un module de marquage selon la présente invention ainsi qu'une cartouche permettant d'ajouter de l'encre dans le réservoir ; et
- la figure 14 illustre un module de marquage selon la présente invention, en cours de nettoyage.

### MODE DE REALISATION DE L'INVENTION

En référence aux figures, l'invention concerne un appareil 10 pour l'application d'un produit colorant, utilisé en particulier pour le maquillage permanent ou le tatouage. Cet appareil est formé essentiellement de deux parties, à savoir un module de marquage 11 comprenant notamment une aiguille 12, et un module d'actionnement 110 de l'aiguille. Selon un mode de réalisation préféré, le module de marquage 11 est amovible de sorte qu'un module de marquage peut être monté sur un module d'actionnement, en être détaché et être remplacé par un autre module de marquage. Dans la pratique, le module d'actionnement 110 comporte au moins un moteur et est utilisé pour réaliser un grand nombre de tatouages alors que plusieurs modules de marquage peuvent généralement être utilisés pour une personne. Pour des raisons d'hygiène, un module de marquage est généralement à usage unique c'est-à-dire qu'il est utilisé pour tatouer une seule personne et qu'il est éliminé après usage sur cette personne.

Le module d'actionnement 110 peut être de n'importe quel type conventionnel et n'est donc ni décrit ni représenté en détails sur les figures. Il est simplement prévu pour recevoir de façon amovible, un module de marquage.

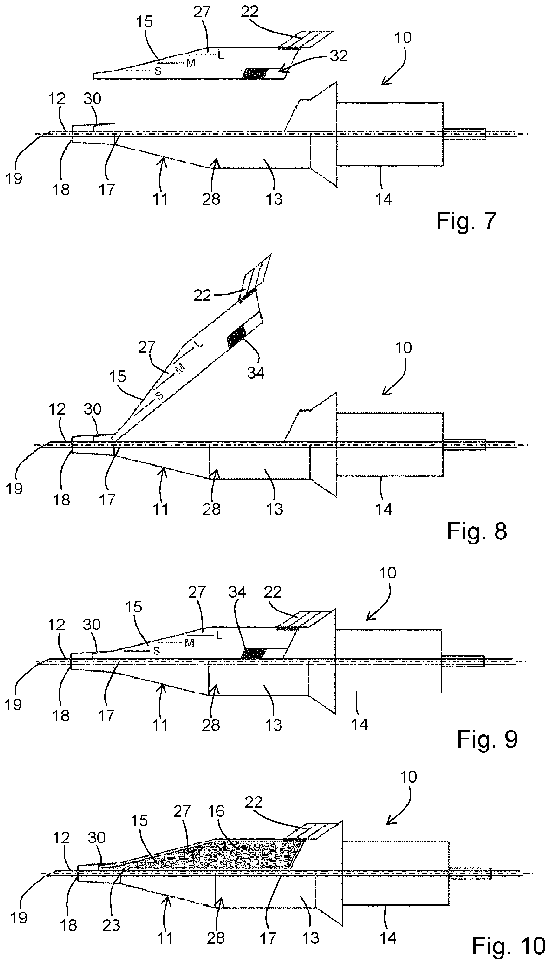

Le module de marquage 11 comporte un corps 28 muni d'une poignée 13 agencée de façon à pouvoir facilement être tenue par l'utilisateur, et un manchon 14 destiné à être introduit dans le module d'actionnement 110. Ce module de marquage 11 comporte en outre l'aiguille 12 mentionnée plus haut et un réservoir 15 de produit colorant tel qu'en particulier de l'encre 16. Ce réservoir peut être fixe et faire partie intégrante du module de marquage ou comporter une cartouche amovible 27 comme cela est représenté par les figures 7 à 14, décrites en détail plus bas.

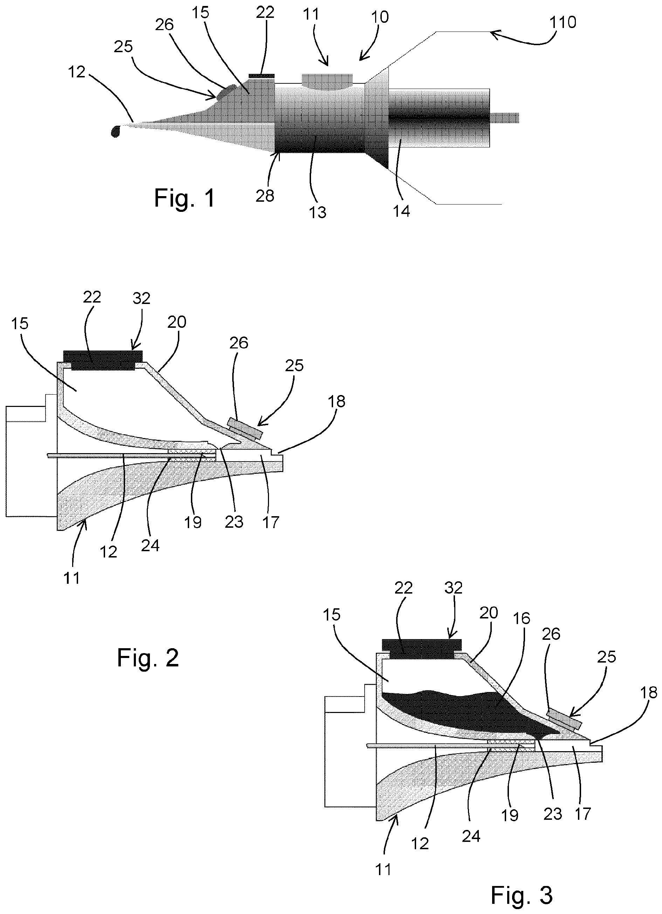

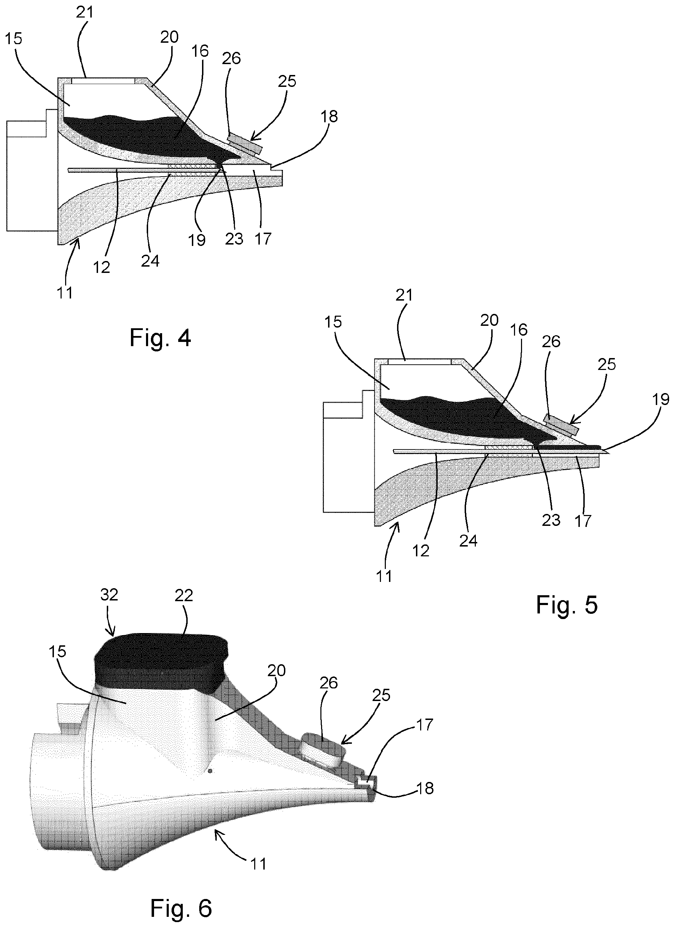

Comme cela est visible en particulier sur les figures 2 à 6, le module de marquage 11 comporte un canal 17 longitudinal dans lequel l'aiguille 12 subit un mouvement alternatif de va-et-vient, sous l'effet du module d'actionnement. Ce canal 17 débouche à une extrémité du module de marquage, dite extrémité libre 18, par laquelle l'aiguille 12 peut dépasser du canal et entrer en contact avec la peau de la personne à tatouer de façon à y déposer de l'encre. Le mouvement alternatif de l'aiguille se fait entre une position rétractée illustrée par la figure 2, dans laquelle une extrémité distale 19 de l'aiguille 12 est entièrement dans le canal 17, et une position étendue illustrée par la figure 5, dans laquelle l'extrémité distale 19 de l'aiguille 12 dépasse du canal 17 et peut entrer en contact avec la peau de la personne sur qui le marquage est réalisé.

Dans le mode de réalisation illustré par les figures 1 à 6, le réservoir d'encre 15 comporte essentiellement une paroi étanche 20, une ouverture de remplissage 21 fermée par un bouchon 22, et une lumière 23 disposée à proximité de l'extrémité libre 18 du canal 17 et reliant l'intérieur du réservoir 15 au canal 17.

Dans le mode de réalisation illustré par les figures 7 à 14, l'encre utilisée pour le marquage ou le tatouage est placé dans la cartouche 27 amovible décrite en détail plus bas et faisant office de réservoir 15.

Lorsque le module de marquage 11 est utilisé selon un mode d'utilisation normal, l'aiguille 12 se déplace longitudinalement dans le canal 17 sous l'effet du module d'actionnement 110. Dans sa position totalement rétractée, c'est-à-dire dans la position dans laquelle l'extrémité distale 19 de l'aiguille 12 est la plus éloignée de l'extrémité libre 18 du canal, la pointe de l'aiguille 12 est en amont de la lumière 23 du réservoir 15. Cette lumière est dimensionnée de façon à laisser passer une faible quantité d'encre 16 sous la forme d'une gouttelette. Lorsque l'aiguille 12 est avancée en direction de l'extrémité libre 18 du canal, elle passe à proximité de la lumière 23 et elle emporte avec elle, la goutelle d'encre 16 qu'elle peut ensuite déposer sous la peau de la personne tatouée, de façon conventionnelle. A chaque cycle de retrait de l'aiguille 12 dans le canal 17 et de déplacement de l'aiguille vers l'avant, à l'extrémité libre 18 du canal 17, l'aiguille 12 prélève une goutelle d'encre 16 provenant du réservoir 15.

Le module de marquage 11 comporte un guidage 24 disposé dans le canal 17. Le guidage 24 s'étend avantageusement sur une zone comprise entre l'endroit où débouche la lumière 23 dans le canal 17 et une zone située en amont de l'endroit où se trouve la pointe ou l'extrémité distale 19 de l'aiguille 12 lorsque cette aiguille est dans sa position totalement rétractée. Ceci présente l'avantage que l'aiguille 12 a toujours une partie contenue dans le guidage 24 et qu'elle est par conséquent toujours guidée de façon adéquate. De plus, le guidage 24 empêche que de l'encre 16 ne soit entraînée en amont de la lumière 23 lorsque l'aiguille 12 effectue un mouvement depuis la position étendue vers la position rétractée. Le guidage 24 peut être disposé par exemple uniquement du côté de la lumière 23 ou être un cylindre percé placé dans le canal, tout autour de l'aiguille.

Selon un premier mode de réalisation du réservoir 15, celui-ci comporte un organe d'amorçage 25 dont la fonction est de pousser une première goutte d'encre 16 dans la lumière 23. Cet organe d'amorçage 25 peut par exemple être formé d'une zone souple dans la paroi étanche du réservoir 15, cette zone souple pouvant être déformée par exemple au moyen d'un bouton 26. La pression sur le bouton 26 génère une surpression à l'intérieur du réservoir, ce qui a pour effet de pousser de l'encre en direction de la zone ouverte du réservoir, à savoir la lumière 23. Lorsqu'une première goutte d'encre 16 a été formée dans la lumière 23, d'autres gouttes se forment par capillarité lorsque la première gouttelette est prélevée par l'aiguille 12.

Selon un deuxième mode de réalisation du réservoir 15, l'ensemble du réservoir 15 peut être formé d'une matière souple, de façon que l'amorçage se fait en pressant n'importe quelle partie du réservoir.

Selon un troisième mode de réalisation, le réservoir 15 comporte une entrée d'air pressurisé, générant une surpression à l'intérieur du réservoir. L'air sous pression peut par exemple être généré par le module d'actionnement dont une partie joue le rôle de compresseur. Dans ce mode de réalisation, il est possible de prévoir un organe de réglage (non représenté) de la pression générée dans le réservoir, le réglage de la pression permettant le réglage du débit d'encre du réservoir dans le canal à travers la lumière. Un tel réglage de pression et/ou de débit est utile pour adapter la quantité d'encre délivrée en fonction du type de trait à réaliser par exemple.

Selon une variante, il est également possible de régler le débit de l'encre 16 en réglant la dimension de la lumière 23. A cet effet, il est possible de réaliser une lumière 23 ayant une zone équipée d'une paroi souple et déformable, cette paroi déformable étant en contact avec une vis de réglage (non représentée) accessible depuis l'extérieur du réservoir ou du module de marquage 11. En l'absence de contrainte, la paroi déformable de la lumière est dans une position telle qu'elle laisse passer un maximum d'encre. Lorsque la vis de réglage est utilisée, elle contraint la paroi déformable de telle façon que la surface transversale de la lumière soit diminuée et que cette lumière laisse passer une moindre quantité d'encre.

Selon une variante, il est possible de remplacer l'arrivée d'air pressurisé dans le réservoir 15 par une aspiration ou une dépression réalisée au niveau du canal. Cette aspiration peut être obtenue au moyen d'une pompe à vide (non représentée) connectée au canal 17, à proximité de son extrémité libre 18. Comme pour le compresseur, la pompe à vide peut être obtenue à partir du module d'actionnement. Le débit d'encre peut également être réglé par un organe de réglage du débit.

Lorsque de l'encre doit être ajoutée dans le réservoir 15 par exemple parce que le contenu de celui-ci a été utilisé, l'encre peut simplement être ajoutée par l'ouverture 21 du réservoir soit en enlevant le bouchon 22 et en versant l'encre 16 dans le réservoir 15, soit en traversant un opercule du bouchon 22, ce qui peut être intéressant pour assurer la stérilité de l'encre. De façon avantageuse, le bouchon 22 du réservoir n'est pas fermé hermétiquement lors de l'utilisation de l'appareil de tatouage, ce qui permet de laisser passer de l'air dans le réservoir au fur et à mesure que de l'encre est consommée.

Selon une variante, le module de marquage de l'invention comporte un mécanisme de rétention 32 de l'encre 16 dans le réservoir 15. Ce mécanisme de rétention 32 est prévu pour empêcher que l'encre ne sorte du réservoir vers le canal 17 lorsque le mécanisme de rétention est activé.

En cas d'activation du mécanisme de rétention 32, l'encre du réservoir n'est pas utilisée. Il est par contre possible d'utiliser de l'encre d'un réservoir externe, ce qui peut être intéressant par exemple lorsqu'une petite partie du tatouage ou du marquage doit être réalisée avec une encre de couleur différente. Lorsque cette partie du tatouage ou du marquage est terminée, le mécanisme de rétention 32 peut être désactivé et l'encre du réservoir peut de nouveau être utilisée. Le mécanisme de rétention est également utile pour le transport et le stockage des cartouches, pour éviter que de l'encre ne sorte de façon involontaire.

Selon un mode de réalisation particulier, le bouchon 22 du réservoir peut prendre trois positions distinctes. Dans une première position, il est ouvert et permet le remplissage de la cartouche 27. Dans une deuxième position, le bouchon 22 est fermé hermétiquement. Dans cette position, de l'air ne peut pas pénétrer dans la cartouche 27 ou le réservoir 15. L'encre ne peut pas s'échapper par la lumière 23 et le réservoir 15 est entièrement fermé. Cette position est représentée par la figure 2. Le bouchon joue le rôle de mécanisme de rétention 32. La position hermétiquement fermée du bouchon correspond à la position activée du mécanisme de rétention.

Dans une troisième position, le bouchon 22 est fermé de façon non hermétique. Ceci permet le passage d'air dans le réservoir 15, ce qui laisse la possibilité à l'encre de s'écouler vers la lumière 23. Cette position est représentée par la figure 3. Elle correspondant à la position désactivée du mécanisme de rétention 32.

Lorsque le bouchon 22 est placé dans sa position non-hermétiquement fermé, le module de marquage 11 peut être utilisé comme décrit précédemment. De l'encre 16 du réservoir 15 traverse la lumière 23 et est prélevée par l'aiguille 12.

Selon une variante du mécanisme de rétention, la course de l'aiguille 12 peut être réglée par le module d'actionnement 110. Dans un premier mode d'utilisation du module de marquage, la course de l'aiguille est comprise entre une première position dans laquelle l'extrémité distale 19 sort du canal 17 du module de marquage et dépasse l'extrémité libre 18 du canal 17, et une deuxième position dans laquelle l'extrémité distale 19 de l'aiguille est en amont de la lumière 23, comme cela est illustré par la figure 3.

Dans un deuxième mode d'utilisation, la course de l'aiguille 12 est comprise entre une première position identique à la première position décrite ci-dessus et une deuxième position dans laquelle l'extrémité distale 19 de l'aiguille 12 est en aval de la lumière 23 et une zone de l'aiguille 12 est toujours en regard de cette lumière 23.

Dans ce cas, la lumière 23 est toujours fermée et l'encre du réservoir ne peut pas s'écouler. De l'encre provenant d'un réservoir externe peut alors être utilisée. Dans ce cas, la course de l'aiguille 12 joue le rôle de mécanisme de rétention 32 de l'encre. Lorsque la course de l'aiguille amène son extrémité distale 19 en amont de la lumière 23, le mécanisme de rétention 32 est désactivé. Lorsque l'extrémité distale 19 de l'aiguille se trouve toujours en aval de la lumière 23, le mécanisme de rétention 32 est activé en ce sens que l'aiguille empêche l'encre de s'écouler par la lumière.

Au lieu de boucher la lumière 23 au moyen de l'aiguille 12 lorsque le mécanisme de rétention 32 est activé, cette lumière 23 pourrait être bouchée par le guidage 24. Ce dernier pourrait en effet être mobile entre une position avancée dans laquelle il bouche la lumière 23 et une position reculée dans laquelle il libère le passage de l'encre dans la lumière.

Les figures 7 à 14 décrivent une variante du module de marquage selon la présente invention. Dans cette variante, l'encre 16 utilisée pour le marquage ou le tatouage est contenue dans un réservoir 15 formé par la cartouche 27 qui est séparable du corps 28 du module de marquage 11. La figure 7 illustre ce module de marquage dans lequel la cartouche d'encre 27 est séparée du corps 28.

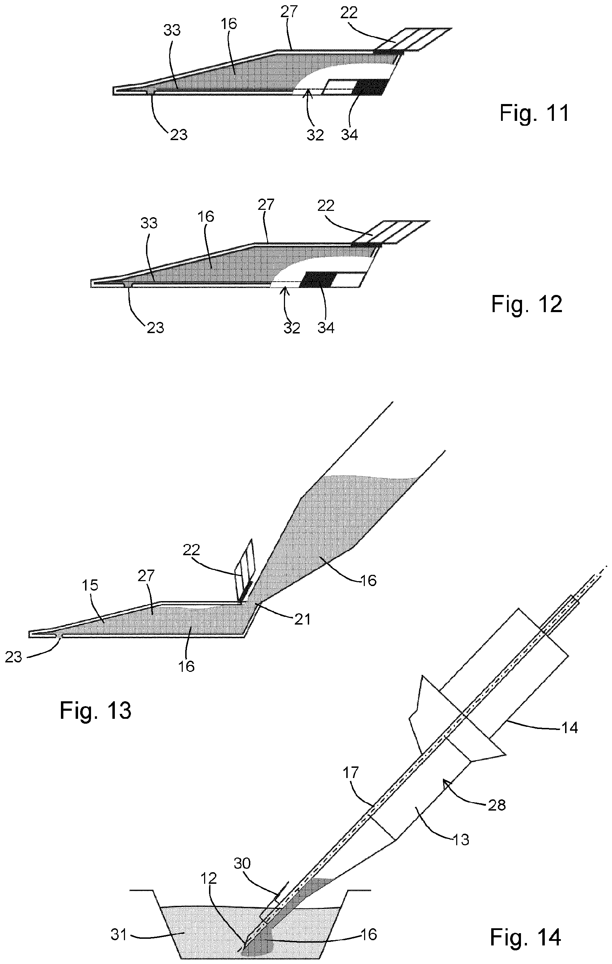

Comme cela est visible en particulier sur les figures 11 et 12, la cartouche 27 comporte une lumière 23 permettant le passage de l'encre de la cartouche au canal 17 du corps ou directement en contact avec l'aiguille 12.

La cartouche 27 comporte en outre un bouchon 22 qui a au moins pour fonction d'ouvrir et/ou de fermer le réservoir 15 en particulier pour remplir la cartouche.

Comme décrit ci-dessus, le bouchon 22 peut prendre trois positions distinctes, à savoir une position ouverte, une position fermée hermétiquement et une position fermée de façon non-hermétique. Il pourrait toutefois également n'avoir que deux positions, à savoir une position ouverte et une position fermée de façon non-hermétique.

La cartouche 27 comporte de préférence un mécanisme de verrouillage (non représenté) permettant de verrouiller cette cartouche en position lorsqu'elle est placée sur le corps 28 du module de marquage. Lorsque le mécanisme de verrouillage est en position déverrouillée, il est possible de retirer la cartouche 27 du corps 28 du module de marquage. Lorsque le mécanisme de verrouillage est en position verrouillée, la cartouche 27 ne peut pas être retirée du corps du module de marquage.

La cartouche 27 peut en outre comporter des graduations et être partiellement transparente de façon à permettre de visualiser et d'évaluer la quantité d'encre contenue dans le réservoir.

Lorsqu'une cartouche 27 doit être mise en place sur le corps 28 d'un module de marquage 11, le mécanisme de verrouillage est positionné dans une position ouverte ou déverrouillée. Une zone de la cartouche 27 est placée dans une zone de retenue 30 du corps du module de marquage, comme cela est illustré par la figure 8, puis la cartouche 27 est placée dans sa position finale, comme illustrée par la figure 9. Le mécanisme de verrouillage est ensuite actionné de façon à verrouiller la cartouche 27 dans sa position d'utilisation.

Lorsque la cartouche 27 est en place, la lumière 23 est placée à proximité de l'aiguille 12. Dans sa position reculée, l'extrémité distale 19 de l'aiguille 12 se trouve en amont de la lumière 23. Lors de son déplacement vers l'avant, l'aiguille prélève une gouttelette d'encre qu'elle peut ensuite déposer sous la peau de la personne à tatouer.

Lorsque la cartouche d'encre 27 doit être remplie, le bouchon 22 est ouvert et de l'encre est introduite par l'ouverture du réservoir 15. La quantité d'entre introduite peut être choisie de façon à correspondre sensiblement à la quantité d'encre nécessaire pour la couleur choisie, ce qui évite le gaspillage.

Le mécanisme de rétention 32 est intégré à la cartouche 27. Ce mécanisme de rétention 32 est illustré plus en détails par les figures 11 et 12 et comporte une languette 33 disposée à l'intérieur de la cartouche 27 et un coulisseau 34 accessible depuis l'extérieur de la cartouche et solidaire de la languette 33. Cette languette est disposée de façon à ce qu'elle puisse se déplacer entre deux postions, à savoir une position ouverte dans laquelle la lumière 23 est entièrement dégagée et la languette 33 n'interfère pas avec cette lumière, et une position fermée dans laquelle la languette 33 bouche la lumière 23 et empêche l'encre de sortir du réservoir à travers la lumière. La position ouverte est illustrée par la figure 11 et la position fermée est illustrée par la figure 12.

Lorsque la languette 33 est en position ouverte ou en d'autres termes, lorsque le mécanisme de rétention 32 est désactivé, de l'encre 16 peut sortir du réservoir 15 par la lumière 23 et le module de marquage 11 peut être utilisé en prélevant l'encre du réservoir 15.

Lorsque la languette 33 est en position fermée ou lorsque le mécanisme de rétention 32 est activé, l'encre ne peut pas sortir du réservoir 15 par la lumière 23. Le module de marquage 11 peut alors être utilisé de la même manière que les modules de marquages conventionnels, à savoir en prélevant de l'encre d'un réservoir externe.

Dans le cas d'un réservoir 15 réalisé sous la forme d'une cartouche 27 amovible, il est possible de lier le mécanisme de verrouillage au mécanisme de rétention d'encre 32, de sorte que le mécanisme de rétention 32 est toujours activé si le mécanisme de verrouillage n'est pas en position verrouillée. Ceci permet d'éviter que de l'encre ne coule et ne sorte du réservoir aussi longtemps que la cartouche n'est pas verrouillée en position sur le corps du module de marquage.

Il est à noter qu'une cartouche 27 peut être placée sur des modules de marquage 11 différents, plus précisément des modules de marquage ayant des aiguilles de forme différentes. Une même cartouche peut par exemple d'abord être placée sur un module de marquage ayant une aiguille spécifique à la réalisation de traits fins, puis la même cartouche peut être placée sur un module de marquage ayant une aiguille spécifique à la réalisation de grandes surfaces et/ou d'ombrages.

L'utilisation de cartouches permet également la mise en place de cartouches 27 préremplies, sans qu'il soit nécessaire que ces cartouches soient remplies par l'utilisateur lui-même.

Par ailleurs, étant donné que l'encre qui est prélevée par l'aiguille 12 passant devant la lumière 23 est déposée sous la peau de l'utilisateur, il n'y a pas d'élément qui soit transmis à « contre-courant », depuis la peau de l'utilisateur à l'encre qui se trouve à l'intérieur de la cartouche. De ce fait, une même cartouche d'encre pourrait être utilisée pour tatouer plusieurs personnes sans qu'il y ait un risque de transmission de pathogène d'une personne à une autre.

Lors d'un changement de couleur notamment, une très faible quantité d'encre peut se trouver sur l'extrémité de l'aiguille 12. L'extrémité du module de marquage à laquelle se trouve l'extrémité distale de l'aiguille peut être placée dans un récipient 31 contenant un liquide de nettoyage, comme cela est illustré par la figure 14. Toute trace d'encre peut alors être éliminée et une nouvelle cartouche d'encre peut être mise en place, par exemple avec une encre de couleur différente. Il n'y a toutefois pas de risque de mélange des encres de couleurs différentes, du fait que seule une très faible quantité d'encre se trouve en contact avec l'aiguille.

Certaines caractéristiques ont été décrites en relation avec les modes de réalisation des figures 1 à 6 et d'autres caractéristiques ont été décrites en relation avec les modes de réalisation des figures 7 à 14. Ces caractéristiques sont par exemple celles concernant l'amorçage de l'encre dans la lumière 23, la position non-hermétiquement fermée du bouchon 22 ou le mécanisme de rétention 32 d'encre. Il est toutefois à noter que ces caractéristiques peuvent également être réalisées sur l'autre mode de réalisation, pour lequel elles ne sont pas spécifiquement décrites.

Contrairement à d'autres appareils de tatouage avec un réservoir d'encre, il n'est pas nécessaire de remplir totalement le réservoir. Il est possible d'ajouter ou d'introduire dans le réservoir, uniquement la quantité d'encre nécessaire.

Si une encre de couleur différente doit être utilisée, selon une première variante, l'encre de la première couleur est sortie du réservoir 15 simplement en ouvrant le bouchon 22 et en vidant le réservoir. Ce dernier peut éventuellement être nettoyé si nécessaire, pour enlever les traces d'encre qui pourraient se trouver contre la paroi du réservoir. Comme l'aiguille 12 prélève de l'encre goutte par goutte, elle n'est pas entièrement recouverte d'encre et le changement de couleur ne nécessite donc pas de nettoyage de l'aiguille.

Selon une deuxième variante, l'encre 16 peut être contenue dans une cartouche amovible. Dans ce cas, la cartouche peut comporter un élément de fermeture qui peut être ouvert pour permettre le passage de l'encre depuis la cartouche amovible jusqu'au canal. Cet élément de fermeture peut par exemple comporter un opercule qui est retiré par l'utilisateur lorsque la cartouche amovible est mise en place ou qui est percé par une pointe disposée à proximité de la lumière 23. L'élément de fermeture peut également être formé par le mécanisme de rétention 32. Ce dernier peut comporter un élément mécanique qui est déplacé lorsque la cartouche est mise en place, de façon à permettre l'écoulement de l'encre de la cartouche au canal 17, et qui bouche le passage entre la cartouche et le canal 17 lorsque la cartouche est retirée du réservoir. Un tel élément mécanique pourrait être une bille ou un clapet par exemple. De cette façon, et du fait que l'aiguille 12 ne se trouve pas dans l'encre, il est simple de changer de couleur d'encre sans changer d'aiguille.

Le mécanisme de rétention 32 peut être activé pour empêcher l'utilisation de l'encre contenue dans la cartouche 27, de façon à permettre l'utilisation d'encre contenue par exemple dans un réservoir externe ou de façon à permettre le stockage et le transport de la cartouche sans risque d'écoulement de l'encre.

Le passage de l'encre peut également être réglé au moyen du bouchon 22. Lorsque celui-ci est placé dans sa position hermétiquement fermée, l'air ne peut pas entrer dans le réservoir et l'encre ne peut pas s'écouler, du fait du faible diamètre de la lumière 23. Lorsque le bouchon 22 est dans sa position non hermétiquement fermée, de l'air peut entrer dans le réservoir et l'encre peut s'écouler par la lumière.

La présente invention permet d'utiliser un appareil de tatouage ou de maquillage permanent sans devoir régulièrement s'arrêter pour ajouter de l'encre sur l'aiguille de tatouage. Il permet de marquer facilement et rapidement des grandes surfaces et de changer de couleur de façon simple. Il peut également être utilisé dans deux modes différents, à savoir un mode dans lequel l'encre du réservoir 15 est utilisée, et un autre mode dans lequel de l'encre provenant d'un réservoir externe est utilisée. La sélection du mode peut se faire rapidement et facilement, en activant ou en désactivant le mécanisme de rétention de l'encre. Le mécanisme de rétention peut bien entendu être utilisé également pour empêcher l'encre de couler, en particulier lorsque le module de marquage n'est pas utilisé et/ou lors du déplacement ou du stockage de cartouches d'encre amovibles.

Par ces différentes caractéristiques, l'appareil est plus facile et plus agréable à utiliser pour la personne qui réalise le maquillage ou le tatouage. Les mêmes opérations peuvent être réalisées en un temps nettement plus court qu'avec les appareils de l'art antérieur ce qui est intéressant pour la personne qui effectue le tatouage, mais surtout pour la personne sur qui le tatouage est réalisé. En effet, un tatouage pouvant être relativement douloureux, un gain de temps lors de la réalisation d'un tatouage implique un plus grand confort pour la personne sur qui le tatouage est réalisé.

La présente invention a été décrite avec un colorant réalisé sous la forme d'une encre liquide. Il est également possible d'utiliser un produit colorant réalisé par exemple sous la forme d'une poudre ou d'une pâte, pour autant que cette poudre puisse être prélevée par l'aiguille lors de son déplacement alternatif dans le canal. Ceci peut notamment être réalisé en générant une surpression dans le réservoir ou une dépression dans le canal, de façon à aspirer la poudre ou la pâte dans ce canal.

## Revendications

1. Module de marquage pour un appareil destiné à l'application d'un produit colorant pour le maquillage permanent ou le tatouage, ce module de marquage (11) comportant un canal (17) et au moins une aiguille (12) mobile longitudinalement dans ledit canal (17) entre une position rétractée dans laquelle une extrémité distale (19) de l'aiguille (12) se trouve à l'intérieur dudit canal (17) et une position étendue dans laquelle l'extrémité distale (19) de l'aiguille (12) dépasse dudit canal (17), ce module de marquage (11) étant **caractérisé en ce qu'**il comporte un réservoir (15) de produit colorant et **en ce que** ledit réservoir (15) communique avec ledit canal (17) uniquement dans une zone située en aval de l'extrémité distale de l'aguille (12) lorsque cette aiguille (12) se trouve dans ladite position rétractée.

2. Module de marquage selon la revendication 1, **caractérisé en ce que** le réservoir (15) comporte une lumière (23) débouchant dans ledit canal (17) dans lequel se déplace l'aiguille (12) et **en ce que** le réservoir (15) communique avec ledit canal (17) par l'intermédiaire de cette lumière (23).

3. Module de marquage selon la revendication 1, **caractérisé en ce que** le réservoir (15) comporte une cartouche amovible (27).

4. Module de marquage selon la revendication 3, comportant un corps (28), **caractérisé en ce que** ce module de marquage (11) comporte un mécanisme de verrouillage (29) pouvant prendre une position de verrouillage dans laquelle la cartouche amovible (27) est maintenue en position sur ledit corps (28), et une position déverrouillée dans laquelle la cartouche amovible (27) peut être séparée du corps (28).

5. Module de marquage selon la revendication 1,**caractérisé en ce qu'**il comporte un mécanisme de rétention (32) de l'encre dans ledit réservoir (15), ce mécanisme de rétention (32) étant agencé pour empêcher l'encre de sortir du réservoir (15) vers le canal (17) lorsque ledit mécanisme de rétention (32) est activé.

6. Module de marquage selon la revendication 1, **caractérisé en ce que** le réservoir (15) comporte un bouchon (22) pouvant prendre une position hermétiquement fermée dans laquelle de l'air ne peut pas rentrer dans le réservoir (15), une position non hermétiquement fermée dans laquelle de l'air peut rentrer dans le réservoir sans que de l'encre puisse être introduite dans le réservoir (15) et une position ouverte dans laquelle de l'air et de l'encre peuvent entrer et sortir du réservoir (15).

7. Module de marquage selon la revendication 6, **caractérisé en ce que** ledit bouchon (22) fait partie du mécanisme de rétention (32), ce mécanisme de rétention (32) étant activé lorsque le bouchon (22) est dans sa position hermétiquement fermée.

8. Module de marquage selon la revendication 5, **caractérisé en ce que** ladite aiguille (12) fait partie du mécanisme de rétention (32), ce mécanisme de rétention (32) étant activé lorsque le déplacement de l'aiguille (12) en direction de sa position rétractée est limité à une position dans laquelle l'extrémité distale (19) de ladite aiguille est toujours en aval de la lumière (23).

9. Module de marquage selon la revendication 5, **caractérisé en ce que** le mécanisme de rétention (32) comporte une languette (33) mobile entre une position fermée dans laquelle cette languette (33) bouche la lumière (23) et une position ouverte dans laquelle la lumière (23) est ouverte.

10. Module de marquage selon la revendication 1, **caractérisé en ce que** le réservoir (15) comporte un organe d'amorçage (25) pour amorcer l'arrivée de produit colorant dans ledit canal (17).

11. Module de marquage selon la revendication 1, **caractérisé en ce qu'**il comporte un dispositif générateur de pression dans le réservoir (15).

12. Module de marquage selon la revendication 1, **caractérisé en ce qu'**il comporte un dispositif générateur de dépression dans le canal (17).

13. Module de marquage selon l'une quelconque des revendications précédentes, **caractérisé en ce qu'**il comporte un organe de réglage du débit d'encre du réservoir (17) dans le canal (17).

14. Module de marquage selon la revendication 1, **caractérisé en ce qu'**il comporte un organe de guidage (24) de l'aiguille, disposé en amont de ladite lumière (23).

15. Appareil pour l'application d'un produit colorant pour le maquillage permanent ou le tatouage, cet appareil (10) étant **caractérisé en ce qu'**il comporte un module de marquage (11) selon l'une quelconque des revendications 1 à 14 et un module d'actionnement (110) de l'aiguille (12) du module de marquage (11).

## Patentansprüche

1. Markierungsmodul für eine Gerät, die zum Auftragen eines Färbemittels für Permanent-Makeup oder Tätowierung bestimmt ist, wobei dieses Markierungsmodul (11) einen Kanal (17) und mindestens eine Nadel (12) umfasst, die in Längsrichtung in dem Kanal (17) zwischen einer eingezogenen Position, in der sich ein distales Ende (19) der Nadel (12) im Inneren des Kanals (17) befindet, und einer ausgefahrenen Position beweglich ist, in der das distale Ende (19) der Nadel (12) aus dem Kanal (17) herausragt, wobei dieses Markierungsmodul (11) **dadurch gekennzeichnet ist, dass** es einen Färbemittelbehälter (15) umfasst und dadurch, dass der Behälter (15) nur in einer Zone mit dem Kanal (17) kommuniziert, die stromabwärts des distalen Endes der Nadel (12) liegt, wenn sich diese Nadel (12) in der eingezogenen Position befindet.

2. Markierungsmodul nach Anspruch 1, **dadurch gekennzeichnet, dass** der Behälter (15) ein Lumen (23) umfasst, das in den Kanal (17) mündet, in dem sich die Nadel (12) bewegt, und dadurch, dass der Behälter (15) mittels dieses Lumens (23) mit dem Kanal (17) kommuniziert.

3. Markierungsmodul nach Anspruch 1, **dadurch gekennzeichnet, dass** der Behälter (15) eine herausnehmbare Patrone (27) umfasst.

4. Markierungsmodul nach Anspruch 3, das einen Körper (28) umfasst, **dadurch gekennzeichnet, dass** dieses Markierungsmodul (11) einen Verriegelungsmechanismus (29) umfasst, der eine Verriegelungsposition, in der die herausnehmbare Patrone (27) am Körper (28) in Position gehalten wird, und eine entriegelte Position einnehmen kann, in der die herausnehmbare Patrone (27) vom Körper (28) getrennt werden kann.

5. Markierungsmodul nach Anspruch 1, **dadurch gekennzeichnet, dass** es einen Mechanismus zum Rückhalten (32) der Tinte im Behälter (15) umfasst, wobei dieser Rückhaltemechanismus (32) so eingerichtet ist, dass er die Tinte daran hindert, aus dem Behälter (15) zum Kanal (17) hin auszutreten, wenn der Rückhaltemechanismus (32) aktiviert ist.

6. Markierungsmodul nach Anspruch 1, **dadurch gekennzeichnet, dass** der Behälter (15) einen Stopfen (22) umfasst, der eine hermetisch geschlossene Position, in der keine Luft in den Behälter (15) eindringen kann, eine nicht hermetisch geschlossene Position, in der Luft in den Behälter eindringen kann, ohne dass Tinte in den Behälter (15) eingeleitet werden kann, und eine offene Position einnehmen kann, in der Luft und Tinte in den Behälter (15) ein- und aus diesem austreten können.

7. Markierungsmodul nach Anspruch 6, **dadurch gekennzeichnet, dass** der Stopfen (22) Teil des Rückhaltemechanismus (32) ist, wobei dieser Rückhaltemechanismus (32) aktiviert wird, wenn sich der Stopfen (22) in seiner hermetisch geschlossenen Position befindet.

8. Markierungsmodul nach Anspruch 5, **dadurch gekennzeichnet, dass** die Nadel (12) Teil des Rückhaltemechanismus (32) ist, wobei dieser Rückhaltemechanismus (32) aktiviert wird, wenn die Bewegung der Nadel (12) in Richtung ihrer eingezogenen Position auf eine Position begrenzt ist, in der sich das distale Ende (19) der Nadel noch stromabwärts des Lumens (23) befindet.

9. Markierungsmodul nach Anspruch 5, **dadurch gekennzeichnet, dass** der Rückhaltemechanismus (32) eine Zunge (33) umfasst, die zwischen einer geschlossenen Position, in der diese Zunge (33) das Lumen (23) verschließt, und einer offenen Position beweglich ist, in der das Lumen (23) offen ist.

10. Markierungsmodul nach Anspruch 1, **dadurch gekennzeichnet, dass** der Behälter (15) ein Startorgan (25) umfasst, um das Ankommen von Färbemittel im Kanal (17) zu starten.

11. Markierungsmodul nach Anspruch 1, **dadurch gekennzeichnet, dass** es eine Druckerzeugungsvorrichtung im Behälter (15) umfasst.

12. Markierungsmodul nach Anspruch 1, **dadurch gekennzeichnet, dass** es eine Unterdruckerzeugungsvorrichtung im Kanal (17) umfasst.

13. Markierungsmodul nach einem der vorstehenden Ansprüche, **dadurch gekennzeichnet, dass** es ein Organ zum Regeln des Durchflusses von Tinte aus dem Behälter (17) in den Kanal (17) umfasst.

14. Markierungsmodul nach Anspruch 1, **dadurch gekennzeichnet, dass** es ein Organ zum Führen (24) der Nadel umfasst, das stromaufwärts des Lumens (23) angeordnet ist.

15. Gerät zum Auftragen eines Färbemittels für Permanent-Makeup oder Tätowierung, wobei dieses Gerät (10) **dadurch gekennzeichnet ist, dass** es ein Markierungsmodul (11) nach einem der Ansprüche 1 bis 14 und ein Modul zum Betätigen (110) der Nadel (12) des Markierungsmoduls (11) umfasst.

## Claims

1. Marking module for an appliance intended for applying a colorant for permanent makeup or tattooing, this marking module (11) having a channel (17) and at least one needle (12) that is able to move longitudinally in said channel (17) between a retracted position in which a distal end (19) of the needle (12) is located inside said channel (17) and an extended position in which the distal end (19) of the needle (12) protrudes from said channel (17), this marking module (11) being **characterized in that** it has a reservoir (15) of colorant and **in that** said reservoir (15) communicates with said channel (17) only in a region situated downstream of the distal end of the needle (12) when this needle (12) is located in said retracted position.

2. Marking module according to claim 1, **characterized in that** the reservoir (15) has a space (23) opening into said channel (17) in which the needle (12) moves, and **in that** the reservoir (15) communicates with said channel (17) by way of this space (23).

3. Marking module according to claim 1, **characterized in that** the reservoir (15) has a removable cartridge (27).

4. Marking module according to claim 3, having a body (28), **characterized in that** this marking module (11) has a locking mechanism (29) being able to take a locking position in which the removable cartridge (27) is held in position on said body (28), and an unlocked position, in which the removable cartridge (27) can be separated from the body (28).

5. Marking module according to claim 1, **characterized in that** it has a mechanism (32) for retaining ink in said reservoir (15), this retaining mechanism (32) being arranged to prevent the ink from exiting the reservoir (15) to the channel (17) when said retaining mechanism (32) is activated.

6. Marking module according to claim 1, **characterized in that** the reservoir (15) has a stopper (22) being able to take a hermetically closed position, in which air cannot enter into the reservoir (15), a non-hermetically closed position, in which air can enter into the reservoir, without ink being able to be introduced into the reservoir (15) and an open position, in which air and ink can enter into and exit from the reservoir (15).

7. Marking module according to claim 6, **characterized in that** said stopper (22) forms part of the retaining mechanism (32), this retaining mechanism (32) being activated when the stopper (22) is in its hermetically closed position.

8. Marking module according to claim 5, **characterized in that** said needle (12) forms part of the retaining mechanism (32), this retaining mechanism (32) being activated when the movement of the needle (12) in the direction of its retracted position is limited to a position, in which the distal end (19) of said needle is always downstream from the space (23).

9. Marking module according to claim 5, **characterized in that** the retaining mechanism (32) comprises a strip (33) that is able to move between a closed position, in which this strip (33) blocks the space (23) and an open position, in which the space (23) is open.

10. Marking module according to claim 1, **characterized in that** the reservoir (15) has a priming member (25) to prime the arrival of the colorant in said channel (17).

11. Marking module according to claim 1, **characterized in that** it has a pressuregenerating device in the reservoir (15).

12. Marking module according to claim 1, **characterized in that** it has a depressiongenerating device in the channel (17).

13. Marking module according to any one of the preceding claims, **characterized in that** it has a member for adjusting the ink flow rate from the reservoir (17) into the channel (17).

14. Marking module according to claim 1, **characterized in that** it has a member (24) for guiding the needle, disposed upstream of said space (23).

15. Appliance for applying a colorant for permanent makeup or tattooing, this appliance (10) being **characterized in that** it has a marking module (11) according to any one of claims 1 to 14 and a module (110) for actuating the needle (12) of the marking module (11).
